Europäisches Patentamt

European Patent Office

Office européen des brevets

(19)

(11) Publication number: **0 200 409 B1**

(12) **EUROPEAN PATENT SPECIFICATION**

(45) Date of publication of patent specification: **29.06.94**

(51) Int. Cl.⁵: **C08L 1/02**, G10K 13/00, D21H 11/00

(21) Application number: **86302755.3**

(22) Date of filing: **14.04.86**

(54) **Moulded material comprising bacteria-produced cellulose.**

(30) Priority: **16.04.85 JP 79291/85**
**07.06.85 JP 122818/85**

(43) Date of publication of application:
**05.11.86 Bulletin 86/45**

(45) Publication of the grant of the patent:
**29.06.94 Bulletin 94/26**

(84) Designated Contracting States:
**DE FR GB**

(56) References cited:
**EP-A- 0 114 481**
**WO-A-86/02095**
**US-A- 4 320 198**
**US-A- 4 378 431**

(73) Proprietor: **AGENCY OF INDUSTRIAL SCIENCE AND TECHNOLOGY**
**3-1, 1-chome, Kasumigaseki**
**Chiyoda-ku Tokyo(JP)**

Proprietor: **SONY CORPORATION**
**7-35 Kitashinagawa 6-chome**
**Shinagawa-ku**
**Tokyo 141(JP)**

Proprietor: **AJINOMOTO CO., INC.**
**5-8, Kyobashi 1-chome, Chuo-ku**
**Tokyo 104(JP)**

(72) Inventor: **Iguchi, Masatoshi**
**No. 3-654, Namiki**
**Sakuramura**
**Niihari-gun Ibaragi-ken(JP)**
Inventor: **Mitsuhashi, Shigenobu**
**No. 2-709, Azuma**
**Sakuramura Niihari-gun(JP)**
Inventor: **Ichimura, Kunihiro**
**No. 5-630, Matsushiro**
**Yatabemachi**
**Tsukuba-gun Ibaragi-ken(JP)**
Inventor: **Nishi, Yoshio**
**No. 6-7-35, Kitashinagawa**
**Shinagawa-ken Tokyo(JP)**
Inventor: **Uryu, Masaru**
**No. 6-7-35, Kitashinagawa**
**Shinagawa-ken Tokyo(JP)**
Inventor: **Yamanaka, Shigeru**
**No. 3-40-13, Ooka**
**Minami-ku**
**Yokohama-shi Kanagawa-ken(JP)**

Inventor: **Watanabe, Kunihiko**
**No. 2-20-8, Kannon**
**Kawasaki-ku**
**Kawasaki-shi Kanagawa-ken(JP)**

(74) Representative: **Bond, Bentley George et al**
**Haseltine Lake & Co.**
**28 Southampton Buildings**
**Chancery Lane**
**London WC2A 1AT (GB)**

**EP 0 200 409 B1**

**Description**

The present invention relates to highly elastic and highly strong moulded materials having excellent tensile strength and stretch resistance which are obtained from cellulose produced by bacteria.

The moulded materials can be utilized not only as paper and as various other sheets but as thread-like mouldings or as various solid mouldings.

As cellulose produced by bacteria, there is known a sheet-like cellulose produced by Acetobacter xylinum ATCC 23769 for medical pads (see JP-A-120159/85).

On the other hand, various materials are known as conventional moulded materials. Thus, cellulose, cellophane, celluloid, etc., obtained by dissolving cellulose derivatives and then finishing, in addition to fibres for forming thread-like mouldings, sheet-like mouldings and various solid mouldings, are known. Furthermore, various synthetic high molecular weight materials have also been developed and include those having particularly improved dynamic strength due to the orientation of the molecular chains in a definite direction.

The dynamic strength of conventional cellulose and cellulose derivatives derived from various plants is not very great. For example, the modulus of elasticity of celluloid or cellophane in sheet form is approximately 2 to 3 GPa at the most.

Furthermore, synthetic high molecular weight materials obtained by orientating the molecular chain in a definite direction have, of necessity, a limited utility due to poor modulus of elasticity in the other directions. This makes these materials useless as strong materials, even though some of then have a modulus of elasticity comparable to that of metals or inorganic materials in one direction. For this reason, materials having no anisotropy in their molecular orientation but having excellent strength as structural materials are desired. However, high molecular weight materials in which the molecules are arranged at random have a poor modulus of elasticity. As moulded materials having a high efficiency, synthetic high molecular weight materials such as polyester films, aramide sheets, polyimide films and the like, are known but the modulus of elasticity thereof is approximately 4 to 7 GPa.

As materials utilizing cellulose produced by bacteria, the foregoing materials are known but their utility is limited to medical pads. Nothing is known as to the use of these materials as high dynamic strength materials.

An object of the present invention is to provide highly elastic and highly strong moulded materials which are excellent in tensile strength and resistance to stretching, as compared to conventional moulded materials.

Another object of the present invention is to provide moulded materials having excellent hydrophilic properties without problem as to toxicity, in addition to the high dynamic strength.

A further object of the present invention is to provide materials having high dynamic strength which are excellent for use in various fields due to the fact that they have imparted thereto electrical conductivity, magnetic properties, highly insulating properties, thermal conductivity, resistance to weather, resistance to chemicals, and so on.

The present inventors have made various investigations with an attempt to attain these objects and found that cellulose comprising ribbon-like microfibrils produced by microorganisms has an extremely large dynamic strength, such as tensile strength, etc., and that the foregoing objects can be achieved by using, as moulded materials, materials having incorporated therein thin bacteria-produced cellulose.

According to the present invention, there is provided a moulded material comprising bacteria-produced cellulose, characterised in that the material has a modulus of elasticity of 10 to 20 GPa, in that the bacteria-produced cellulose comprises ribbon-like microfibrils having a width of 10 to 50 nm (100 to 500 Angstroms) and a thickness of 1 to 20 nm (10 to 200 Angstroms), and in that the material has been produced by pressing a gel of the bacteria-produced cellulose at a squeezing pressure of 1 to 10 kg/cm$^2$.

The present invention also provides an acoustic diaphragm characterised in that it is made from a material having a modulus of elasticity of 10 to 20 GPa, which material comprises bacteria-produced cellulose comprising ribbon-like microfibrils having a width of 10 to 50 nm (100 to 500 Angstroms) and a thickness of 1 to 20 nm (10 to 200 Angstroms).

The content of bacteria-produced cellulose of the material of the invention may be from 0.01 to 100%.

Preferably, the material additionally contains at least one material selected from (a) a hydrophilic high molecular weight material, (b) a hydrophobic high molecular weight material, (c) a metal, (d) an inorganic material, (e) a substance which is magnetic, (f) a substance which is electrically conductive, (g) a substance having a high thermal conductivity, (h) a substance having a high resistance to weathering, and (i) a substance having high resistance to chemicals.

3

The bacteria-produced cellulose is readily decomposed by cellulose to form glucose. For example when cellulose (EC 3.2.1.4) (manufactured by Amono Pharmaceutical Co., Ltd.) was dissolved in a 0.1% (w/v) suspension of the cellulose in an amount of 0.5% (w/v) and the mixture was reacted at 30°C for 24 hours in a 0.1M acetate buffer, it was observed that a part of the cellulose decomposed. The supernatant was developed by paper chromatography, whereby cello-oligosaccharides and glucose were detected. In addition, a small quantity of hexoses, other than glucose, were detected.

Thus, the bacteria-containing cellulose of the present invention contains cellulose, and it may contain heteropolysaccharides having cellulose as a main chain and $\beta$-1,3, $\beta$-1,2-, etc., glucans.

In the case of heteropolysaccharides, the constituent components, other than cellulose, are hexose, pentose and organic acids, etc., such as mannose, fructose, galactose, xylose, arabinose, ramnose, glucuronic acid, etc. These polysaccharides may be single substances; alternatively, two or more polysaccharides may be combined via, for example, a hydrogen bond, etc.

Any bacteria-produced cellulose is usable in the present invention.

Microorganisms that produce such cellulose are not particularly limited, and include <u>Acetobacter aceti subsp. xylinum</u> ATCC 10821, <u>Acetobacter pasteurianus</u>, <u>Acetobacter rancens</u>, <u>Sarcina ventriculi</u>, <u>Bacterium xylinside</u> and bacteria belonging to the genus <u>Pseudomonas</u>, the genus <u>Agrobacterium</u>, etc.

The methods for culturing these microorganisms and accumulating bacteria-produced cellulose are conventional methods for culturing bacteria. Thus, microorganisms are inoculated on conventional nutrient media containing carbon sources, nitrogen sources, inorganic salts and, if necessary, organic trace nutrients such as amino acids, vitamins, etc. followed by settling or gentle aerial shaking. As carbon sources, glucose, sucrose, maltose, starch hydrolysates, molasses, etc., may be used but ethanol, acetic acid, citric acid, etc., may also be used singly or in combination with the above-desribed sugars. As nitrogen sources, organic or inorganic nitrogen sources such as ammonium salts, e.g. ammonium sulphate, ammonium chloride, ammonium phosphate, etc., nitrates, urea, peptone or the like may be used. Inorganic salts, phosphates, magnesium salts, calcium salts, iron salts, manganese salts, etc., may be used. As organic trace nutrients, amino acids, vitamins, fatty acids, nucleic acids, etc., may be used. Furthermore, peptone, casamino acid, yeast extracts, soybean protein hydrolysates, etc., containing these nutrients may be used. When using auxotrophs requiring amino acids, etc., for growth, it may be necessary to use further nutrients.

The culture conditions may be conventional; for example, at a pH of 3 to 9 and at a temperature of 20 to 40°C, culture may be performed for 1 to 30 days, whereby the bacteria-produced cellulose accumulates as a surface layer in a gel form.

The gel may be withdrawn and washed with water, if necessary. Depending upon the intended use of the gel, the washing water may contain chemicals such as sterilizers, pre-treating agents, etc.

After washing with water, the gel may be dried or kneaded with other materials, for use.

It is preferred that the bacteria-produced cellulose be of structure in which the microfibrils are intertwined, in order to enhance the dynamic strength such as tensile strength, etc. For this reason, a method which for example, comprises pressing the gel, withdrawn from the culture, from the orthogonal direction, squeezing most of the free water off and then drying it, is effective. It is appropriate that the squeezing pressure be approximately 1 to 10 kg/cm$^2$. By this press squeezing, the cellulose after drying is orientated along the press squeezing direction. Furthermore, by stretching in one direction while applying pressure, e.g. by performing a rolling operation, the cellulose after drying is orientated also in the rolling direction, in addition to the press squeezing direction. Pressing apparatuses can be appropriately chosen from commercially available machines.

On the other hand, it is also effective to macerate the bacteria-produced cellulose, to increase the dynamic strength. Maceration may be carried out by using a mechanical shearing force. The bacteria-produced cellulose can easily be macerated with, for example, a rotary macerator, a mixer, etc. It is also effective to conduct the aforesaid press squeezing after maceration.

The moulded material having a high dynamic strength of the present invention can be in the form of various shapes such as sheet-liked shapes, yarn-like shapes, cloth-like shapes, solid-like shapes, etc.

In the case of moulding into a sheet-like form, the bacteria-produced cellulose is, if desired, macerated and then formed into a layer, which is squeezed under pressure, if desired, and then dried. By press squeezing, a planar-orientated sheet is obtained; by further rolling, a sheet not only planar-orientated but also uniaxially orientated can be obtained.

It is desired that the drying of the sheet, macerated and/or press squeezed, be carried out after fixing it to a suitable support. By fixing it on a support, the degree of planar-orientation is further enhanced and a sheet having a large dynamic strength can be obtained. As supports, plates, e.g. glass plates, metal plates, etc., having, for example, a net structure, can be used. Any drying temperature can be used as long as the temperature is within a range where the cellulose is not decomposed. In addition to heat drying, freeze drying can also be used.

The thus obtained sheet has a structure in which the microfibrils are intertwined at random. According to an X-ray diffraction pattern, the press-squeezed sheet is planarily orientated; the additionally rolled sheet is planarily orientated and, at the same time, uniaxially orientated. The modulus of elasticity of the sheet is generally about 10 to 20 GPa.

The thickness of the sheet depends upon its intended use, but is generally about 1 to 500 microns.

The sheet may contain various additives. For example, by incorporating solutions (aqueous or non-aqueous), emulsions, dispersions, powders, melts, etc. of various high molecular weight materials, one or more of strength, weatherproofness, chemical resistance, water resistance, water repellency, antistatic properties, etc., can be imparted to the sheet, depending upon the properties of the additives. By incorporating metals such as aluminium, copper, iron, zinc, etc., or carbon in a powdery form or fibre form, electroconductivity and thermal conductivity can be increased. Further by incorporating inorganic materials such as titanium oxide, iron oxides, calcium carbonate, kaolin, bentonite, zeolite, mica, alumina, etc., the heat resistance, insulating properties, etc., can be improved or smoothness can be imparted to the surface, depending upon kind thereof. By incorporating lower molecular weight organic materials or adhesives, the strength can be further increased. The sheet may be coloured with colouring agents such as phthalocyanine, azo compounds, indigos, safflowers, etc. For colouration, various paints, dyes and pigments can be used in addition thereto. By incorporating medicines or sterilizers, the sheet can also be utilized as a medical sheet.

These kneadins and additives are incorporated in an appropriate amount not exceeding 97% by imparting the desired physical properties. The time of the incorporation is not limited, and they may be incorporated in the bacteria-produced cellulose gel or a macerated product thereof; alternatively, they may be incorporated after press squeezing or after drying. Furthermore, they may be incorporated in the media or culture on in some occasions. The method of incorporation may be by impregnation, in addition to mixing.

On such a sheet can also be laminated a layer of other material. The laminate can be appropriately chosen depending upon the intended purpose of the sheet. The laminate can also be chosed from the aforesaid kneadings or additives; for example, various high molecular weight materials can be coated onto the sheet to impart waterproofness to the sheet.

In the case of paper, the bacteria-produced cellulose can be macerated, then subjected to paper making and then drying, whereby paper having an excellent tensile strength, resistance to expansion, etc., and at the same time having a high elasticity and a high strength and which is chemically stable and excellent in water absorbance and air permeability can be obtained. In this case, ordinary additives, treating agents, etc., used for paper making can be utilized and kneadings and additives can also be appropriately chosen from the aforesaid substances and incorporated into the paper.

In recent years, the demand for electrically insulating paper, heat resistant paper, fire retarding paper, etc., has increased and synthetic paper, inorganic paper, etc., containing-non-cellulose fibres have been prepared. When one attempts to prepare such paper by a wet method, it is necessary to perform paper making by the use of cellulose pulp, because non-cellulose fibres fail to form hydrogen bonds except where pulp fibres such as fibres of polyethylene, polypropylene, polyacrylonitrile, aromatic polyamide, etc., are used. In this case, it is necessary that the amount of pulp be minimized as much as possible to enhance the insulating, heat resistant and fire retarding properties. In the case of mixing conventional wood pulp with paper, however, the amount added reaches 20 to 50% and the intended effect cannot be sufficiently achieved. On the contrary, the amount of cellulose can be greatly reduced by the use of the bacteria-produced cellulose instead of wood pulp and paper having excellent insulating, heat resistance and fire retarding properties can be obtained. Accordingly, the moulded material having a high dynamic strength of the present invention is also effective in this respect.

Furthermore, it is said that photo-crosslinking polyvinyl alcholol has a good affinity to living things as compared to conventional photo-crosslinking resins and it is thought that the utility of immobilizing agents for enzymes, microorganisms, etc. would be further improved. A photoresist used for making an original printing plate can be prepared by coating a resin on a base plate, by projecting a design, etc., to be printed thereon to cause photo-crosslinking and hardening of the resin, and by washing off unhardened resin. The photo-crosslinkable polyvinyl alcohol, which is water-soluble, has advantages in that it is inexpensive and the washing thereof is easy as compared to conventional oil-soluble photoresists, and is expected to be useful is a photoresist. In this case, however, there is a problem in that photo-crosslinkable polyvinyl alcohol swells in water and the cross-linked structure is destroyed. However, this swelling can be prevented by incorporating bacteria-produced cellulose into it.

In the case of yarn, for example, the bacteria-produced cellulose gel or macecated product thereof may be washed, dried and then dissolved in, for example, a dimethylacetamide/lithium chloride solvent. The solution is spun using a coagulating solution such as water, alcohols, ketones, dioxane, tetrahydrofuran, etc., in which cellulose is insoluble and the solvent for the cellulose is miscible.

In the case of cloth, this yarn may be woven in a conventional manner.

In the case of a solid structure, various plastics materials may be kneaded with or laminated on the bacteria-produced cellulose to form the desired moulding. This moulding is substitutable for, example, various fibre-reinforced plastics products or carbon fibre products.

The bacteria-produced cellulose comprises ribbon-like microfibrils and has large dynamic strengths such as tensile strength, expansion resistance, elasticity, etc. The dynamic strength is enhanced by the microfibrils heing intertwined. By imparting orientation thereto, the strength in such a direction is further increased. The bacteria-produced cellulose has the property that is readily orientated by pressing.

The moulded material having a high dynamic strength of the present invention is excellent in tensile strength, expansion resistance, elasticity, etc. In particular, the sheet obtained after press squeezing and drying has an extremely high modulus of elasticity; in the case of the articles of the Examples hereinbelow, the modulus of elasticity was more than twice that of the sheet of polymetaphenyleneisophthalamide having the highest modulus of elasticity among secondary materials heretofore known.

Accordingly, the material can be used as reinforcing material for composite plastics materials requiring a high strength, for example, as a body material for ships, aircrafts, automobiles, etc., as a printed circuit base, etc., as a high quality paper such as recording paper, etc., or as diaphragm, etc., for percussion instruments.

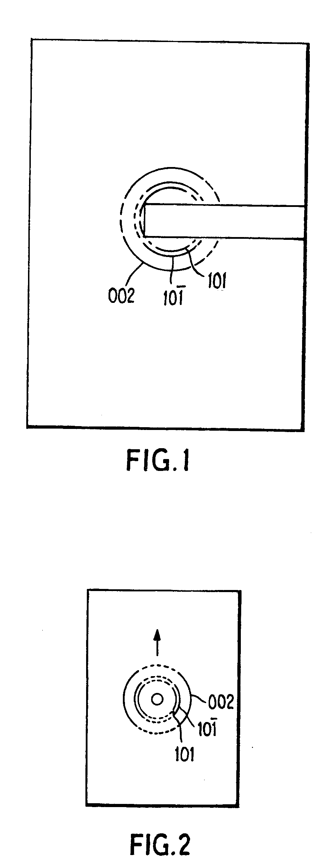

The invention will now be illustrated by the following Examples, in which reference is made to Figures 1 to 4 of the drawings, which Figures are explained in the relevant Examples.

The bacteria-produced cellulose used in the Examples was derived from <u>Acetobacter</u> <u>aceti</u> <u>subsp.</u> <u>xylinum</u> ATCC 10821 (which is available to the public, from the American Type Culture Collection).

The following abbreviations are used in the Examples:

N.U.SP : soft wood unbleached sulphite pulp

CSF : Canadian standard Freeness (TAPPI Bulletin T 227 M-58, May 1948)

L.B.KP : hard wood pulp bleached kraft paper.

Example 1

In a 200 ml Erlenmeyer flask, there was charged 50 ml of a medium consisting of 5 g/dl of sucrose, 0.5 g/dl of yeast extract (Difco), 0.3 g/dl of $KH_2PO_4$ and 0.05 g/dl of $MgSO_4.7H_2O$, and having a pH of 5.0. The medium was sterilized with steam at 120°C for 20 minutes. The sterilized medium was innoculated with a platinum loop of <u>Acetobacter</u> <u>aceti</u> <u>subsp.</u> <u>xylinum</u> ATCC 10821, grown at 30°C for 30 days in a test tube slant agar medium (pH 6.0) consisting of 0.5 g/dl of yeast extract, 0.3 g/dl of peptone and 2.5 g/dl of mannitol (pH 6.0), and the medium was cultured at 30°C. Thirty days thereafter, a gel-like membrane containing white bacteria-produced cellulose polysaccharides had formed as a surface layer on the culture solution.

The thus obtained gel-like membrane was washed with water and spread in a layer having a thickness of about 1 cm. By pressing the membrane under a pressure of about 10 kg/cm$^2$ using a test pressing machine (made by Tester Industry Co., Ltd.), water was squeezed out. The layer was attached to a glass plate and dried at 105°C for 2 hours to obtain a sheet having a thickness of about 10 microns.

The X-ray diffraction pattern of the sheet thus obtained is shown in Figure 1. This Figure shows the diffraction pattern obtained by having its rotation axis parallel to the sheet plane and photographing with an X-ray incident upon the direction orthogonal to the rotation axis. As shown in the Figure, plane 101, plane 101, and plane 002 are all orientated, and this sheet undergoes planar orientation to an extremely high degree.

The modulus of elasticity of this sheet, of known cellulosic sheets and of various high molecular weight secondary materials were measured using a tensile tester. The results are given in Table 1.

Table 1

| Sheet | Modulus of Elasticity |
|---|---|
| This invention | 15.8 Gpa |
| Cellophane | 1.5 |
| Celluloid | 2.0 |
| Nomex* | 7.0 |
| Lumilar** | 4.9 |

*: Sheet of polymetaphenylene isophthalamide
**: Sheet of biaxially orientated polyethylene terephthalate

Example 2

Gel-like bacteria-produced cellulose, as used in Example 1, was squeezed under pressure while rolling it in one direction using a roll pressing machine (made by Yoshida Kogyo K.K.). In the same manner as Example 1, the squeezed cellulose was attached to a glass plate and dried at 105°C for 2 hours to obtain a sheet.

The X-ray diffraction pattern of the sheet thus obtained is shown in Figure 2. This Figure shows the diffraction pattern obtained by fixing the sheet and photographing it with an X-ray incident upon the direction perpendicular to the film surface. In the Figure, the arrow indicates the direction of rolling. As shown in the Figure, orientation is found in each of plane 101, plane 101 and plane 002, and uniaxial orientation is obviously clear. Furthermore, with respect to planar orientation, orientation almost similar to that of Figure 1 is clear.

The modulus of elasticity of this sheet was measured in a manner similar to Example 1, and was found to be 20 GPa in the rolling direction.

Example 3

Bacteria-produced cellulose was incorporated in novolid fibres (Kainol Fiber KF 0203, 14 microns in diameter and 3 mm in length, manufacutred by Gun-Ei Chemical Industry Co., Ltd.) and a sheet having a weight of 60 g/m$^2$ was produced therefrom by paper making according to the TAPPI method (TAPPI standard T 205 m-58).

Furthermore, for the purpose of comparison, papier mache was made from ordinary wood pulp (N.U.SP), beaten to a high degree (CSF 245 ml), and Kainol Fiber.

The breaking length of each of these sheets was measured using an automatic recording tensile tester. The results are given in Table 2.

Table 2

|  |  |  |  |  |  | Breaking Length |
|---|---|---|---|---|---|---|
| Kainol | 95 parts | B.C.* | 5 parts | 0.33 km |
| " | 90 parts | " | 10 parts | 0.79 km |
| " | 80 parts | " | 20 parts | 1.67 km |
| Kainol | 90 parts | N.U.SP | 10 parts | 0.12 km |

*B.C.:  bacteria-produced cellulose

By the use of bacteria-produced cellulose, paper making is possible and the strength of the paper is increased, by the use of the bacteria-produced cellulose in a small amount. In the case of using conventional pulp, paper making was impossible in the case of the use of less than 10 parts.

Example 4

Using various inorganic fibres and bacteria-produced cellulose, papier mache was prepared, and the breaking length was measured. The results are given in Table 3.

## Table 3

| | | Breaking Length |
|---|---|---|
| Carbon Fibre (Toray, Toreka T 088, 3mm long) | 95 parts | } 0.15 km |
| Bacteria-produced cellulose | 5 parts | |
| Carbon Fibre (same as above, 6mm long) | 90 parts | } 0.64 km |
| Bacteria-produced cellulose | 5 parts | |
| Alumina Fibre (made by Denka, Arecen Bulk) | 90 parts | } 0.24 km |
| Bacteria-produced cellulose | 5 parts | |

In each case, paper making was possible in the case of the use of 5 to 10% of the bacteria-produced cellulose.

Example 5

Gel-like bacteria-produced cellulose was pressed and dried to obtain a sheet. The Young's modulus (E) of the sheet, measured by the oscillation lead method, was 13.6 GPa.

This value is 5 to 10 times the Young's modulus of conventional paper prepared from wood pulp alone.

Example 6

Gel-like bacteria-produced cellulose was macerated with a homogenizer, and the macerated material was made into paper according to the TAPPI method. The Young's modulus (E) of the sheet, measured by the oscillation lead method, was 7.4 GPa.

Furthermore, for the purpose of improving the freeness and yield of fine particles, 5% (ratio of solids contents) of polyamide epichlorohydrin resin (Kaimen 557H, manufactured by Dick Hercules Co., Ltd.) was incorporated therein, and paper was made therefrom. The Young's modules (E) of this paper was 8.1 GPa.

In each case, paper having a high strength was obtained.

8

Example 7

To bacteria-produced cellulose, there was added wood pulp (N.U.KP) (CSF, 540 ml). Furthermore, 5% (ratio of solids contents) of aluminium sulphate was added to the mixture, and paper was made from the mixture. The properties of the paper thus obtained were as given in Table 4.

### Table 4

N.U.KP   100 parts   Breaking Length   3.69 km

N.U.KP   80 parts  
B.C.     20 parts   }   "   4.81 km

N.U.KP   100 parts   E= 1.38 GPa

N.U.KP   80 parts  
B.C.     20 parts   }   E= 2.16 GPa

By the addition of the bacteria-containing cellulose, the strength of the paper was improved.

Example 8

After macerating gel-like bacteria-produced cellulose, obtained by definite culture, with a standard pulp macerator, the macerated material was filtered using a 125 mesh sieve to obtain macerated paste having a solids content of about 8.8%. The paste was used in the following experiments.

Photo-crosslinkable polyvinyl alchol, (PVA)-SbQ (GH-17SbQ, 10.5 wt%, 1.2 mol%, manufactured by Toyo Gosei Kyogyo K.K.), the macerated paste described above and water were mixed in a dark room in the ratios given in Table 5.

Table 5

| Sample | (a) | (b) | (c) |
|--------|------|------|------|
| 1 | 21.0 | 6.0 | 7.5 |
| 2 | 21.0 | 1.8 | 11.7 |
| 3 | 21.0 | 0.6 | 12.9 |
| 4 | 21.0 | 0 | 13.5 |
| (a) PVA-SbQ (GH-17 SbQ, 10.5 wt%, 1.2 mol%) | | | |
| (b) macerated bacteria-produced cellulose (calculated as dry weight) | | | |
| (c) $H_2O$ | | | |

Each mixture given in the Table above was spread over an acrylic plate in a thickness of about 0.7 mm, using a glass rod. After air drying overnight, each mixture was further air dried at 40°C for 30 minutes. These operations were performed in a dark room. Each mixture was exposed to sunlight for 30 minutes to cause cross-linking and to obtain a sheet.

The sheet was cut into a ribbon-like shape of 3 x 1 cm and put into water for 2 hours to measure the extent of swelling. The results are given in Table 6.

Table 6

| Sample | Length x Width (cm) | | Swelling Rate by Length | Weight x $10^2$ (g) | | Swelling by Rate by Weight |
|---|---|---|---|---|---|---|
| | 0h | 2h | | 0h | 2h | |
| 1 | 3x1 | 3.2x1.1 | 1.07 | 2.46 | 4.87 | 1.98 |
| 2 | 3x1 | 3.3x1.1 | 1.10 | 1.18 | 2.56 | 2.17 |
| 3 | 3x1 | 3.3x1.1 | 1.10 | 1.43 | 3.34 | 2.36 |
| 4 | 3x1 | 4.0x1.3 | 1.33 | 1.02 | 2.63 | 2.50 |

By the use of the bacteria-produced cellulose, swelling could be prevented.

Next, tensile tests were performed on the sheets. The results are given in Table 7.

Table 7

| Sample | Modulus of Tensile Elasticity (GPa) |
|---|---|
| 1 | 1.61 |
| 2 | 1.71 |
| 3 | 1.32 |
| 4 | 1.01 |

By using the bacteria-produced cellulose, the modulus of elasticity could be improved to 1.3 to 1.6 times.

Example 9

Each of the mixtures given in Example 8 was sandwiched between two glass plates using as a spacer a slide glass having a thickness of 1 mm. Each mixture was then exposed to sunlight for 30 minutes to cause crosslinking and a gel similar to konjak (an edible gel made from plant material) in a wet state was obtained. The gel was put into distilled water and a swelling test was performed in a manner similar to Example 8. The results are given in Table 8.

Table 8

| Sample | Weight (g) | | Ratio of Water Absorbancy |
|---|---|---|---|
| | 0 h | 2 h | |
| 1 | 1.75 | 3.97 | 2.3 |
| 2 | 0.90 | 3.95 | 4.4 |
| 3 | 0.48 | 5.05 | 10.05 }* |
| 4 | 1.74 | 40.3 | 23.2 } |

*Samples 3 and 4 were destroyed by swelling.

By using the bacteria-produced cellulose, swelling and destruction of the gel were prevented.

Example 10

To 3.5 parts of dry bacteria-produced cellulose, there were added 100 parts of dimethylacetamide. The mixture was refluxed with stirring for 60 minutes. Next, the mixture was cooled to 100°C. After slowly adding 10 parts of lithium chloride, the mixture was stirred at room temperature overnight to dissolve the bacteria-produced cellulose. The resulting spinning dope was spun in a tetrahydrofuran coagulating solution, at a spinning draft of 1.5 and at a bath length of 80 cm. The yarns from the spinning bath were stretched by 50% in water add at 50°C and then dried. The properties of the fibres thus obtained are given in Table 9. For the purpose of comparison, wood pulp (L.B. KP) was spun in a similar manner.

Table 9

|  | Dry Strength (g/d) | Wet Strength (g/d) | Dry Strength (%) | Wet Strength (%) |
|---|---|---|---|---|
| BC | 5.4 | 4.6 | 8.1 | 12.3 |
| L.B.KP | 3.2 | 2.1 | 5.7 | 7.9 |

Example 11

To a mixture of copper powder (manufactured by Fukuda Kinzokuhakufun Kogyo K.K., 10 microns in diameter) and wood pulp (N.U. KP) (CSF, 540 ml), macerated bacteria-produced cellulose was added, and the mixture was subjected to paper making by the TAPPI method. Furthermore, for the purpose of comparison, papier mache was also prepared from copper powder and wood pulp. The physical properties thereof were measured using an automatic recording tensile tester. The results are given in Table 10.

$$\underline{Table\ 10}$$

| | Stretching % | Strength (kg/mm$^2$) | Modulus of Elasticity (kg/mm$^2$) |
|---|---|---|---|
| Copper powder 100 parts<br>N.U.KP 10 parts<br>BC 5 parts | 1.6 | 0.68 | 56 |
| Copper powder 100 parts<br>N.U.KP 10 parts | 1.6 | 0.23 | 31 |

By the use of the bacteria-produced cellulose, the copper powder did not egress and the strength of the paper was greatly improved. In the case of the conventional pulp, more than 60% of the copper powder egressed.

Example 12

To wood pulp (N.U.KP) (CSF, 540 ml), there was added macerated bacteria-produced (BC), and the mixture was subjected to paper making by the TAPPI method. The resulting paper was impregnated with a phenolic resin and air dried. By hot press finishing, a phenolic laminated plate was prepared. Furthermore, for the purpose of comparison, a phenolic laminated plate comprising wood pulp alone was also prepared in a similar manner. These phenolic laminated plates were moulded into Dumbell Moldel No. 1 (JIS K-7113) and the physical properties thereof were measured using an automatic recording tensile teeter. The results are given in Table 11.

## Table 11

| | Stretching % | Strength (kg/cm$^2$) | Modulus of Elasticity (kg/mm$^2$) |
|---|---|---|---|
| N.U.KP 95 parts / BC 5 parts | 5.2 | 808 | 4400 |
| N.U.KP 100 parts | 4.4 | 658 | 3300 |

By the use of the bacteria-produced cellulose, the strength of the phenolic laminated plate was greatly improved.

### Example 13

Gel-like bacteria-produced cellulose as used in Example 1 was subjected to hot pressing (using a machine made by Yoshida Kogyo K.K.) at 150°C under a pressure of 5 kg/cm$^2$ for 5 minutes to obtain a sheet. A polyethylene-imine-treated polyethylene film was laminated onto the thus obtained sheet at 320°C to prepare a laminated film. The physical properties of the film were measured using an automatic recording tensile tester. The laminate film had a greatly improved modulus of elasticity, i.e. 16.2 GPa, as compared to ordinarly cellophane-polyethylene laminated film, which had a modulus of elasticity of 1.7 GPa.

### Example 14

To silicon nitride and silicon carbide (manufactured by Tateho Chemical Co., Ltd., 10 microns in length) there was added macerated bacteria-produced cellulose (BC), and the mixture was subjected to paper making by the TAPPI method. Furthermore, for the purpose of comparison, papier mache made of wood pulp (N.U.KP) and microfibril cellulose (MFC, manufactured by Daicel Chemical Co., Ltd,) was also treated in a similar manner. The physical properties of the sheets obtained were measured using an automatic recording tensile tester. The results are given in Table 12.

Table 12

|  | | Modulus of Elasticity $(kg/mm^2)$ |
|---|---|---|
| Silicon nitride 100 parts<br>BC 5 parts | } | 15 |
| Silicon nitride 100 parts<br>BC 3 parts | } | 12 |
| Silicon nitride 100 parts<br>BC 10 parts | } | 35 |
| Silicon nitride 100 parts<br>N.U.KP 5 parts | } | 8 |

By the use of the bacteria-produced cellulose, no egression of silicon nitride and silicon carbide occurred but the modulus of elasticity, was greatly improved. In the case of ordinary pulp, 60% or more of silicon nitride and silicon carbide egressed. In the case of MFC, most of the silicon nitride and silicon carbide egressed.

Example 15

In a 500 ml Sakaguchi flask, there was charged a 50 ml aliquot of a liquid medium consisting of 2 g/dl of fumaric acid, 0.2 g/dl of dihydrogen potassium phosphate, 1 mg/dl of magnesium sulphate tetrahydrate, 1 mg/dl of magnesium sulphate heptahydrate, 0.05 g/dl of calcium chloride, 1.0 g/dl of yeast extract (Difco) and 1.0 g/dl of peptone (Difco), which medium was adjusted to a pH of 7.0 using ammonia. One platinum loop of E. coli ATCC 11775 was inoculated on the medium followed by culturing at 30°C for 24 hours. Then, the bacteria were recovered by centrifugation in a conventional manner. After washing twice with physiological saline, the bacteria were suspended in the same amount of physiological saline as their wet weight.

By the following method, an attempt was made to immobilize the bacteria on a carrier prepared from gelatin and cellulose. As the method for immobilization, the method using transglutaminase, as described-in JP-A-66886/84, was used. Gelatin (supplied by Miyagi Kagaku) and the macerated cellulosic substance were mixed in ratios given in Table 13. To the mixture, the bacteria were added in an amount of 3.5% and the mixture was gelled in the manner described in JP-A-66886/84. Thus, 0.1 unit was added per 1 mg of transglutaminase. The mixture was allowed to stand at 25°C for 1 hour to cause gellation. The gel was cut into cubes 5 mm square and added to a reaction mixture to examine the aspartase activity. The reaction mixture contained 20 g/dl of fumaric acid and 1mm of $MgSO_4.7H_2O$, and the pH was adjusted to 8.5 using ammonia. Bacteria-immobilized gelatin get was added to the reaction mixture in a bacteria concentration of 0.5% based on the total amount of the reaction mixture. A reaction was carried out for 1 hour. Every 5 minutes the concentration of aspartic acid was quantitatively assayed by ninhydrin colorimetry to determine the initial reaction rate. The count of the bacteria egressed into the reaction mixture was determined by a colony count after 30 minutes from the initiation of the reaction. The results are given in Table 13.

Table 13

| Concentration of Gelatin (%) | Reinforcement and Amount Added (%) | Count of Bacteria (cells/ml) |
|---|---|---|
| 12.5 | 0 | $1.2 \times 10^8$ |
| 12.0 | Macerated bacteria-produced cellulose 0.5 | $1.1 \times 10^7$ |
| 12.5 | Macerated bacteria-produced cellulose 0.5 | $9.8 \times 10^6$ |
| 12.0 | Polyester gauze 0.5 | $1.4 \times 10^8$ |
| 12.5 | Polyester gauze 0.5 | $1.1 \times 10^8$ |

The enzyme activity was determined from the initial reaction rate. The enzyme activity was expressed by a relative value, taking as 100 the case of the addition of no cellulosic substance. Further the reaction was repeated 10 times. The results are given in Table 14.

Table 14

| Concentration of Gelatin (%) | Reinforcement and Amount Added (%) | Relative Activity of Aspartase | |
|---|---|---|---|
| | | 1st | 10th |
| 12.5 | 0 | 100 | 35 |
| 12.0 | present cellulosic substance 0.5 | 99 | 80 |
| 12.5 | present cellulosic substance 0.5 | 102 | 85 |
| 12.0 | polyester gauze 0.5 | 103 | 37 |
| 12.5 | polyester gauze 0.5 | 97 | 38 |

By the incorporating the cellulosic substance, the egression of the bacteria was prevented so that it became possible to maintain the activity after repeated use.

Furthermore, the breaking strength of gel supplemented with gelatin and reinforcing materials similar to those given in Table 13 was measured. The measurement was performed by expressing as a breaking strength the maxium load obtained by causing gellation of the aforesaid gelatin solution in a cylindrical plastic container having a diameter of 22 cm, then setting it in a rheometer (Fudo Kogyo K.K., NRM 2002 J) and immersing an adapter of 5 mm diameter directly into the gel. The results are given in Table 15.

Table 15

| Concentration of Gelatin (%) | Reinforcement and Amount Added (%) | Breaking Strength (%) |
|---|---|---|
| 12.5 | 0 | 90 |
| 12.5 | present cellulosic substance 0.5 | 145 |
| 12.5 | present cellulosic substance 0.5 | 194 |
| 12.0 | polyester gauze 0.5 | 111 |
| 12.5 | polyester gauze 0.5 | 128 |

With the cellulosic material, a reinforcing effect superior to that of a conventional reinforcement was noted.

Example 16

Immobilization to alginic acid gel was conducted by mixing sodium alginate with the cellulosic substance according to Table 16. Thereafter the mixture was added dropwise to a 0.1M $CaCl_2$ solution to obtain a bead-like gel.

An aspartase reaction was carried out under the same reaction conditions as used in Example 15. The count of bacteria egressed was determined by the number of colonies after 30 minutes, without exchanging the reaction mixture. The results are given in Table 16.

14

Table 16

| Concentation of Alginic Acid (%) | Reinforcement and Amount Added (%) | Count of Bacteria Egressed after 30 Minutes (cells/ml) |
|---|---|---|
| 1.5 | 0 | $2.1 \times 10^8$ |
| 1.0 | present cellulosic substance 0.5 | $5.3 \times 10^6$ |
| 1.5 | present cellulosic substance 0.5 | $1.5 \times 105$ |
| 1.0 | polyester gauze 0.5 | $1.9 \times 10^8$ |
| 1.5 | polyester gauze 0.5 | $2.0 \times 10^8$ |

The reaction solution described above was replenished every 15 minutes, and the reaction of the immobilized carrier was carried out 4 times in total. The enzyme activity was determined from its initial rate by measuring the concentration of aspartic acid every 3 minutes. The results are given in Table 17. The aspartase activity is shown by a relative value, the value first obtained when no cellulosic substance was added being 100. The results are given in Table 17.

Table 17

| Concentration of Aspartic Acid (%) | Reinforcement and Amount Added (%) | Relative Activity of Aspartase | | | |
|---|---|---|---|---|---|
| | | 1 | 2 | 3 | 4 |
| 1.5 | 0 | 100 | 85 | 32 | 20 |
| 1.0 | present cellulosic substance 0.5 | 101 | 92 | 79 | 60 |
| 1.5 | present cellulosic substance 0.5 | 97 | 90 | 87 | 85 |
| 1.0 | polyester gauze 0.5 | 101 | 80 | 29 | 19 |
| 1.5 | polyester gauze 0.5 | 98 | 89 | 39 | 25 |

As a result of using the cellulosic substance as a reinforcement, the egression of the bacteria was prevented so that it became possible to repeatedly use the immobilized carrier, having the high strength of wood pulp as compared to the conventional immobilized carrier.

Example 17

In the following manner, invertase was immobilized onto a photo-crosslinkale resin. A mixture of 1 part of invertase and 2 parts of phosphate buffer (pH 6.0) was mixed with 20 parts of a photo-crosslinkable resin solution (pH 6.0). The mixture was spread over a glass plate. After air drying for 1 day, irradiation with light was performed for 1 hour to cause crosslinking and hardening. The cellulosic substance was incorporated in a final concentration of 5%.

The immobilized membrane was cut into a size of 5 x 5 mm and reacted in 50 ml of a solution of 4 g of sucrose at 40°C for 24 hours with stirring to examine the decomposition rate of sucrose. The reaction was repeated 10 times. Furthermore, the breaking stress and modulus of elasticity were also examined. The results are given in Table 18.

Table 18

| Reinforcement and Amount Added (%) | Breaking Stress (MPa) | Modulus of Elasticity (GPa) | Relative Activity of Invertase | |
|---|---|---|---|---|
| | | | 1st | 10th |
| 0 | 25.1 | 0.95 | 100 | 98 |
| Present cellulosic substance 5 | 32.0 | 1.53 | 100 | 99 |
| Polyester gauze 5 | 28.3 | 1.20 | 100 | 98 |

By incorporation the cellulosic substance into the membrane, the physical properties of the membrane such as the breaking shear, modulus of elasticity, etc., representing the strength of the immobilized enzyme membrane, were greatly improved. For this reason, the immobilization of the enzyme was easy and the time required for the immoblization in the case of the use of the cellulosic substance was shortened to 75% as compared to the case in which no cellulosic substance was used.

Example 18

In a 200 ml Erlenmeyer flash, there was charged 50 ml of a medium (pH 5.0) consisting of 5 g/dl of sucrose, 0.5 g/dl of yeast extract, 0.5 g/dl of ammonium sulphate, 0.3 g/dl of hydrogen potassium phosphate ($KH_2PO_4$) and 0.05 g/dl of magnesium sulphate ($MgSO_4.7H_2O$). The medium was sterlized with steam at 120°C for 20 minutes to form a culture solution.

Then, the culture solution was inoculated with one platinum loop of Acetobacter aceti subsp. xylinum - (ATCC 10821), grown at 30°C for 3 days in a test tube slant agar medium (pH 6.0) consisting of 0.5 g/dl of yeast extract, 0.3 g/dl of peptone and 2.5 g/dl of mannitol, and the culture solution was cultured at 30°C for 30 days under the conditions described above whereby a gel-like membrane containing white bacteria-produced cellulose polysaccharides was formed in the upper layer of the culture solution. This gel-like membrane of cellulosic polysaccharides was washed with water to obtain bacteria-produced cellulose.

The thus obtained bacteria-produced cellulose was inserted between metal plates and press dried at 130°C to obtain a sheet of bacteria-produced cellulose.

The physical properties of the bacteria-produced cellulose sheet were measured by the oscillation lead method. The results given in Table were obtained.

Table 19

| | |
|---|---|
| Young's Modulus | 13.6 GPa |
| Density | 1060 kg/m$^2$ |
| Resonance sharpness Q | 29.2 |
| Sonic velocity | 3580 m/sec. |

Then, a paper honeycomb was prepared using the sheet of bacteria-produced cellulose as a skin agent. A core having a cell size of 3 mm, which had been immersed in 6% phenol and which had a thickness of 2.8 mm, was used.

A flat speaker, 60 mm square, was prepared using the paper honeycomb as a diaphragm. This is designated Sample 1.

For the purpose of comparison, a paper honeycomb was prepared using the same core as Sample 1 and kraft paper as a skin agent. Using the paper honeycomb as a diaphragm, a flat speaker was prepared in a manner similar to Sample 1, which speaker is designated Comparative Sample 1. The physical properties of the kraft paper used herein are shown in Table 20.

Table 20

| | |
|---|---|
| Young's modulus | 2.4 GPa |
| Density | 565 kg/m$^2$ |
| Resonance sharpness Q | 35.1 |
| Sonic velocity | 2060 m/sec. |

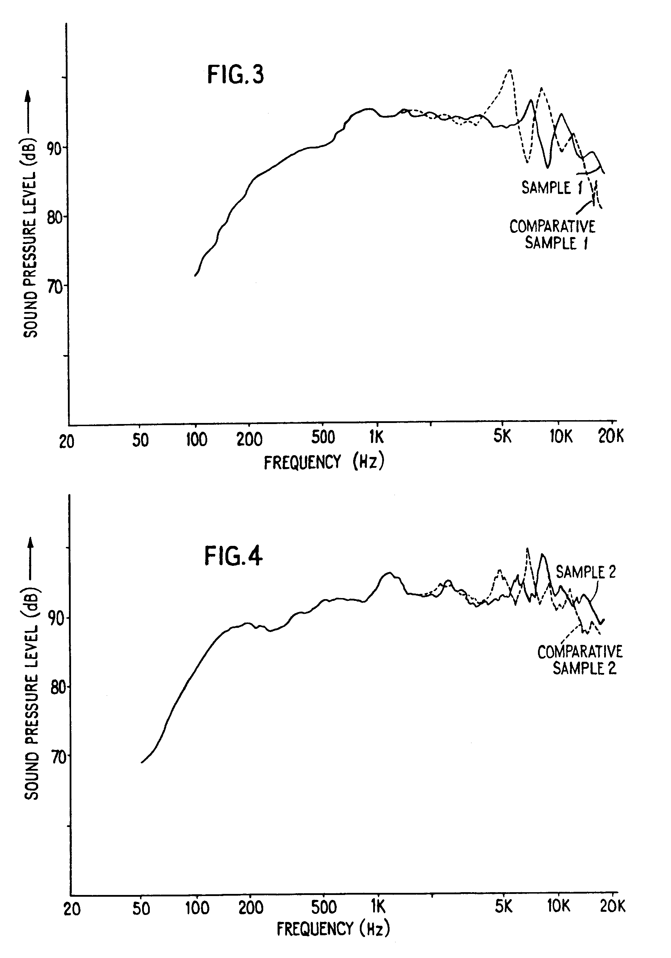

The frequency characteristics of Sample 1 and Comparative Sample 1 were measured. The results are shown in Figure 3.

From Figure 3, it is noted that the high band reproducing threshold frequency is shifted to a high frequency band and, at the some time, this peak becomes small in the case of Sample 1, using the bacteria-produced cellulose sheet as the skin agent, and thus the reproducing frequency band is greatly broadened.

16

Example 19

The bacteria-produced cellulose prepared in the foregoing Example was macerated in a macerator. After adding 4% (ratio of solids contents) of rosin size and 4% (ratio of solids contents)of aluminium sulphate as sizing agents, cone paper was prepared by a conventional paper making process. The physical properties of the cone paper were measured by the oscillation lead method. The results given in Table 21 were obtained.

Table 21

| Young's modulus | 6.3 GPa |
| Density | 982 kg/m$^2$ |
| Resonance sharpness Q | 21.0 |
| Sonic velocity | 2530 m/sec. |

Then, a full range speaker unit having a diameter of 12 cm was prepared using this cone paper. This is designated Sample 2.

For the purpose of comparison, a full range speaker unit was prepared in a manner similar to Sample 2, using cone paper prepared from ordinary kraft pulp, which unit is designated Comparative Sample 2. The physical properties of the cone paper are given in Table 22.

Table 22

| Young's modulus | 2.5 GPa |
| Density | 620 kg/m$^2$ |
| Resonance sharpness Q | 21.0 |
| Sonic velocity | 2010 m/sec. |

The frequency characteristics of Sample 2 and Comparative Sample 2 were measured. The results are shown in Figure 4.

From Figure 4, it is noted that, in the case of Sample 2 prepared from cone paper containing the bacteria-produced cellulose, the high band reproducing threshold frequency was shifted to a high frequency and and the reproducing frequency band was broadened.

Example 20

A part of the wood pulp used for making ordinary cone paper was replaced (in amounts of 5% and 15%) by the bacteria-produced cellulose obtained in Example 18, as shown in Table 23, and paper making by the TAPPI method was carried out. Thus, cone paper (Samples 3 and 4) was obtained. As the wood pulp, N.U.SP (freeness, 570 ml, Csf) was used, and the bacteria-produced cellulose used was macerated bacteria-produced cellulose. Furthermore, cone paper obtained by paper making using wood pulp alone was made, and is designated Comparative Sample 3.

The physical properties of these samples were measured by the oscillation lead method. The results are given in Table 23.

Table 23

| | Young's Modulus (GPa) | Density (kg/m$^2$) | Resonance Sharpness (Q) | Sonic Velocity (m/sec) |
|---|---|---|---|---|
| Sample 3 | 1.55 | 486 | 20.2 | 1790 |
| Sample 4 | 1.79 | 503 | 21.3 | 1880 |
| Comparative Sample 3 | 1.38 | 454 | 21.2 | 1750 |

From Table 23, it is noted that by using the bacteria-produced cellulose, an improvement in strength is obtained while internal loss is maintained.

17

As is apparant from the present Example and from Example 18 and 19, in the acoustic diaphragm of the present invention, the bacteria-produced cellulose is used at least as part of the cellulose fibres thereof, and a paper diaphragm having an extremely high strength and a broadened reproducing frequency band is obtained.

Furthermore, in the case of the diaphragm of the present invention, there is no danger of problems such as a reduction of internal loss, a sense of incompatibility in sound quality, etc., because it is unnecessary to use non-cellulosic materials (which are unable to form hydrogen bonds).

**Claims**

1. A moulded material comprising bacteria-produced cellulose, <u>characterised in that</u> the material has a modulus of elasticity of 10 to 20 GPa, <u>in that</u> the bacteria-produced cellulose comprises ribbon-like microfibrils having a width of 10 to 50 nm (100 to 500 Angstroms) and a thickness of 1 to 20 nm (10 to 200 Angstroms), and <u>in that</u> the material has been produced by pressing a gel of the bacteria-produced cellulose at a squeezing pressure of 1 to 10 kg/cm$^2$.

2. A material according to claim 1, wherein said bacterial-produced cellulose is produced by a microorganism belonging to the genus <u>Acetobacter</u>.

3. A material according to claim 1 or 2, wherein said cellulose is orientated along the press squeezing direction.

4. A material according to any of claims 1 to 3, prepared by a process comprising an impregnating step of said bacteria-produced cellulose.

5. A material according to any of claims 1 to 3, prepared by a process comprising a coating step of said bacteria-produced cellulose.

6. A material according to any of claims 1 to 5, in the form of a sheet.

7. A material according to claim 6, wherein said sheet is paper.

8. A process for producing a moulded material comprising bacteria-produced cellulose, <u>characterised in that</u> the material has a modulus of elasticity of 10 to 20 GPa, <u>in that</u> the bacteria-produced cellulose comprises ribbon-like microfibrils having a width of 10 to 50 nm (100 to 500 Angstroms) and a thickness of 1 to 20 nm (10 to 200 Angstroms), and <u>in that</u> the material is produced by pressing a gel of the bacteria-produced cellulose at a squeezing pressure of 1 to 10 kg/cm$^2$.

9. An acoustic diaphragm <u>characterised in that</u> it is made from a material having a modulus of elasticity of at least 10 GPa, which material comprises bacteria-produced cellulose comprising ribbon-like microfibrils having a width of 10 to 50 nm (100 to 500 Angstroms) and a thickness of 1 to 20 nm (10 to 200 Angstroms).

10. An acoustic diaphragm according to claim 9, wherein the material has been macerated.

11. An acoustic diaphragm according to claim 9 or 10, wherein the material is blended with at least one material selected from (a) a hydrophilic high molecular weight material, (b) a hydrophobic high molecular weight material, (c) a metal, (d) an inorganic material, (e) a substance which is magnetic, (f) a substance which is electrically conductive, (g) a substance having a high thermal conductivity, (h) a substance having a high resistance to weathering, and (i) a substance having a high resistance to chemicals.

12. An acoustic diaphragm according to any of claims 9 to 11, wherein the material has been prepared by a process comprising an impregnating step of said bacteria-produced cellulose.

13. An acoustic diaphragm according to any of claims 9 to 11, wherein the material has been prepared by a process comprising a coating step of said bacteria-produced cellulose.

EP 0 200 409 B1

**Patentansprüche**

1. Geformtes Material, das bakteriell gebildete Cellulose enthält, dadurch gekennzeichnet, daß das Material einen Elastizitätsmodul von 10 bis 20 GPa hat, daß die bakteriell gebildete Cellulose bandförmige Mikrofibrillen einer Breite von 10 bis 50 nm (100 bis 500 Angström) und einer Dicke von 1 bis 20 nm (10 bis 200 Angström) umfaßt, und daß das Material durch Pressen eines Gels der bakteriell gebildetem Cellulose bei einem Quetschdruck von 1 bis 10 kg/cm$^2$ gebildet worden ist.

2. Material nach Anspruch 1, wobei die bakteriell gebildete Cellulose durch einen Mikroorganismus des Genus Acetobacter produziert worden ist.

3. Material nach Anspruch 1 oder 2, wobei die Cellulose längs der Richtung des Quetschdruckes orientiert ist.

4. Material nach einem der Ansprüche 1 bis 3, das mit Hilfe eines Verfahrens hergestellt worden ist, welches eine Stufe des Imprägnierens der bakteriell gebildeten Cellulose einschließt.

5. Material nach einem der Ansprüche 1 bis 3, das mit Hilfe eines Verfahrens hergestellt worden ist, das eine Stufe des Beschichtens der bakteriell gebildeten Cellulose einschließt.

6. Material nach einem der Ansprüche 1 bis 5 in Form einer Folie.

7. Material nach Anspruch 6, wobei die Folie Papier ist.

8. Verfahren zur Herstellung eines geformten Materials, das bakteriell gebildete Cellulose enthält, dadurch gekennzeichnet, daß das Material einen Elastizitätsmodul von 10 bis 20 GPa hat, daß die bakteriell gebildete Cellulose bandartige Microfibrillen einer Breite von 10 bis 50 nm (100 bis 500 Angström) und einer Dicke von 1 bis 20 nm (10 bis 200 Angström) aufweist, und daß das das Material durch Pressen eines Gels der bakteriell gebildeten Cellulose bei einem Quetschdruck von 1 bis 10 kg/cm$^2$ hergestellt wird.

9. Akustische Membran, dadurch gekennzeichnet, daß sie aus einem Material mit einem Elastizitätsmodul von mindestens 10 GPa hergestellt worden ist, wobei das Material bakteriell gebildete Cellulose umfaßt, die bandartigen Mikrofibrillen von einer Breite von 10 bis 50 nm (100 bis 500 Angström) und einer Dicke von 1 bis 20 nm (10 bis 200 Angström) aufweist.

10. Akustische Membran nach Anspruch 9, wobei das Material dem Mazerieren unterworfen worden ist.

11. Akustische Membran nach Anspruch 9 oder 10, wobei das Material mit mindestens einem Material vermischt worden ist, das ausgewählt ist unter (a) einem hydrophilen hochmolekularen Material, (b) einem hydrophoben hochmolekularen Material, (c) einem Metall, (d) einem anorganischen Material, (e) einer magnetischen Substanz, (f) einer elektrisch leitfähigen Substanz, (g) einer Substanz mit hoher Wärmeleitfähigkeit, (h) einer Substanz mit hoher Bewitterungsfestigkeit und (i) einer Substanz mit hoher Chemikalienbeständigkeit.

12. Akustische Membran nach einem der Ansprüche 9 bis 11, wobei das Material mit Hilfe eines Verfahrens hergestellt worden ist, das eine Stufe des Imprägnierens der bakteriell gebildeten Cellulose einschließt.

13. Akustische Membran nach einem der Ansprüche 1 bis 11, wobei das Material mit Hilfe eines Verfahrens hergestellt worden ist, das eine Stufe des Beschichtens der bakteriell gebildeten Cellulose einschließt.

**Revendications**

1. Matière moulée comprenant de la cellulose produite par des bactéries, caractérisée en ce que la matière a un module d'élasticité de 10 à 20 GPa, en ce que la cellulose produite par des bactéries comprend des microfibrilles en rubans ayant une largeur de 10 à 50 nm (100 à 500 angströms) et une

19

épaisseur de 1 à 20 nm (10 à 200 angströms), et en ce que la matière a été produite par pressage d'un gel de la cellulose produite par des bactéries à une pression d'essorage de 1 à 10 kg/cm².

2. Matière selon la revendication 1, dans laquelle ladite cellulose produite par des bactéries est produite par un microorganisme appartenant au genre *Acetobacter*.

3. Matière selon la revendication 1 ou 2, dans laquelle ladite cellulose est orientée le long de la direction de l'essorage sous pression.

4. Matière selon l'une quelconque des revendications 1 à 3, préparée par un procédé comprenant une étape d'imprégnation de ladite cellulose produite par des bactéries.

5. Matière selon l'une quelconque des revendications 1 à 3, préparée par un procédé comprenant une étape de revêtement de ladite cellulose produite par des bactéries.

6. Matière selon l'une quelconque des revendications 1 à 5, sous forme d'une feuille.

7. Matière selon la revendication 6, dans laquelle ladite feuille est du papier.

8. Procédé de production d'une matière moulée comprenant de la cellulose produite par des bactéries, caractérisé au que la matière a un module d'élasticité de 10 à 20 GPa, en ce que la cellulose produite par des bactéries comprend des microfibrilles en rubans ayant une largeur de 10 à 50 nm (100 à 500 angströms) et une épaisseur de 1 à 20 nm (10 à 200 angströms), et en ce que la matière est produite par pressage d'un gel de la cellulose produite par des bactéries à une pression d'essorage de 1 à 10 kg/cm².

9. Diaphragme acoustique, caractérisé en ce qu'il est fabriqué à partir d'une matière ayant un module d'élasticité d'au moins 10 GPa, cette matière contenant de la cellulose produite par des bactéries comprenant des microfibrilles en rubans ayant une largeur de 10 à 50 nm (100 à 500 angströms) et une épaisseur de 1 à 20 nm (10 à 200 angströms).

10. Diaphragme acoustique selon la revendication 9, dans lequel la matière a été macérée.

11. Diaphragme acoustique selon la revendication 9 ou 10, dans lequel la matière est mélangée avec au moins une matière choisie parmi (a) une matière de masse moléculaire élevée hydrophile, (b) une matière de masse moléculaire élevée hydrophobe, (c) un métal, (d) une matière inorganique, (e) une substance magnétique, (f) une substance conductrice de l'electricité (g) une substance ayant une conductivité thermique élevée, (h) une substance ayant une résistance élevée aux intempéries, et (i) une substance ayant une résistance élevée aux produits chimiques.

12. Diaphragme acoustique selon l'une quelconque des revendications 9 à 11, dans lequel la matière a été préparée par un procédé comprenant une étape d'imprégnation de ladite cellulose produite par des bactéries.

13. Diaphragme acoustique selon l'une quelconque des revendications 9 à 11, dans lequel la matière a été préparée par un procédé comprenant une étape de revêtement de ladite cellulose produite par des bactéries.

FIG.1

FIG.2

FIG.3

SOUND PRESSURE LEVEL (dB)

90

80

70

SAMPLE 1

COMPARATIVE
SAMPLE 1

20    50    100   200   500   1K        5K    10K   20K
FREQUENCY (Hz)

FIG.4

SOUND PRESSURE LEVEL (dB)

90

80

70

SAMPLE 2

COMPARATIVE
SAMPLE 2

20    50    100   200   500   1K        5K    10K   20K
FREQUENCY (Hz)

22